# EUROPEAN PATENT APPLICATION

(11) **EP 0 589 452 A1**
(43) Date of publication of application: **30.03.1994**
(21) Application number: 93115354.8
(22) Date of filing: 23.09.1993
(51) Int. Cl.: A61B 17/34, A61M 25/04

(54) **A tissue gripping apparatus for use with a cannula or trocar assembly**

(30) Priority: 23.09.1992 US 950434
(71) Applicant: United States Surgical Corporation, Norwalk, Connecticut 06856 (US)
(72) Inventor: Green, David T., Westport, CT 06880 (US); Tovey, Jonathan H., Milford, CT 06460 (US); Smith, Robert C., Watertown, CT 06795 (US)
(74) Representative: Marsh, Roy David

(57) **Abstract**

A tissue gripping apparatus (10) is provided including a cylindrical member (12) positioned about a tubular body portion or cannula (24). The cylindrical member (12) includes a plurality of articulated arms (16) that are movable between engagable and non-engagable positions. A hinge (18) proximal the midpoint in the articulated arms (16) enhances the tissue gripping abilities of the apparatus (10). The apparatus (10) also includes an external sealing device (40) having a flange (44) a distal end such that the flange (44) is positionable against a patient's body. The flange (44) provides a sealing effect such that gases from the body cavity will not escape, for example, during surgical procedures requiring insufflation. The sealing device (40) may also provide enhanced stabilization of a cannula positioned through an incision in the body cavity.

## Description

### BACKGROUND OF THE INVENTION

### 1. Field of the Invention

The present invention relates to surgical instruments for performing laparoscopic and endoscopic surgical procedures, and more particularly to a device for simultaneously securing instruments such as cannulas in an incision in a patient's body during the surgical procedure while preventing, leakage of gases around the exterior of a cannula particularly after extensive instrument manipulation.

### 2. Discussion of the Prior Art

In recent years, laparoscopic and endoscopic surgical procedures have become increasingly popular for performing major surgical operations. In such a procedure, a small incision or puncture is made in the patient's body to provide access for a tube or a cannula device. The tube or cannula device is inserted into the patient's body to allow for the insertion of instruments used in performing the surgical procedure, as well as for the insertion of a camera or endoscope to view the surgical objective. Typically, a trocar device including, for example, an obturator and a cannula is employed to puncture the skin and provide access to the surgical area. A pointed obturator may be used for penetrating the skin to extend the trocar beyond the body wall to the surgical site. Alternatively, an incision may be made using a scalpel or similar device before inserting a blunt obturator through the incision. When either obturator is removed, the cannula remains in place to maintain access to the surgical site, and several incisions may be made to provide numerous access ports to the surgical objective. Once the cannulas are in place, various surgical instruments such as scissors, dissectors, retractors or the like, may be inserted by a surgeon to perform the surgery. Typically, a scope device is used to view the area directly, or a miniature camera is used to display the surgical site on a video monitor in the operating room.

The primary benefit of such minimally invasive surgical techniques is the reduction of scarring, and consequently, minimizing damage to surrounding tissue and organs. As a result, recovery time is greatly reduced for the patient.

During a laparoscopic surgical procedure, gas is introduced into the body cavity, usually the abdomen, by means of a pneumoperitoneum needle. The gas inflates the abdominal cavity to provide greater access to the surgical area and minimize obstruction during surgery. The trocar assembly is then inserted into the body cavity to a point adjacent the tissue or organ which is the surgical objective. Due to the gas insufflation, it is necessary to maintain a desired gas seal at each of the cannulas in position in the body. After the obturator is removed from the trocar assembly, the cannula remains in place in the patient's body. Ordinarily, a flapper valve in a housing at the proximal end of the cannula prevents the insufflation gas from escaping through the cannula after the obturator is removed. However, gas leakage may also occur from around the incision where the cannula has been positioned. Further, the routine movement of the cannula during the surgical procedure may result in a loss of gas tightness about the cannula. More specifically, axial, lateral and/or angular movement of the cannula may result in gas leakage from around the incision, and thereby, negatively affect the surgical procedure. It is also preferred to maintain the cannulas in a relatively stable state, primarily to free the surgeon and the surgical assistants from having to hold the cannulas to prevent these instruments from backing off and consequently being displaced from the incision.

In order to support the cannula in a hands-off manner, and maintain the integrity of the gas seal at the incision, it has been known to provide various mechanisms and devices which attempt to maintain and secure the cannula in the incision.

Typical devices include penetration limiting devices such as the sleeve or collar disclosed in U.S. Patent No. 3,817,251 to Hasson, which provides a conical sleeve which may be adjusted to various positions on the cannula to limit insertion of the cannula to specific depths. U.S. Patent No. 4,077,412 to Moossun, U.S. Patent No. 4,637,814 to Leiboff, as well as U.S. Patent No. 4,627,838 to Cross et al., disclose devices to prevent the inadvertent removal or backing off of the cannula during the surgical procedure. Moossun provides an inflatable diaphragm member which is inflated once the cannula is positioned in the body cavity to prevent inadvertent removal of the cannula from the incision until the diaphragm is deflated. Leiboff also discloses an inflatable cuff member which is inflated in a body cavity to secure the device and seal the cavity at the opening to prevent leakage of irrigating fluid during an irrigation procedure. Cross et al. provide a complex wing-type mechanism which is extended once the catheter is positioned within the body cavity so that the wing members engage the body wall to prevent removal of the catheter until the wing mechanism is collapsed. These devices often require additional equipment to be present in the operating room, such as a gas or compressed air source to inflate the device.

Also known are sleeve members having external ribs which fit over a cannula. The sleeve is forced into the incision either by twisting or simply by forcing the sleeve into the incision along with the catheter. Further, devices are known that include a "Malecott" type wing arrangement such as U.S. Patent 4,608,965 to Anspach, Jr. et al. Anspach discloses an endoscopic retainer and tissue retracting device including a cylindrical tube having flexible strips at a distal end. The device is placed over an endoscope and the retracting device's flexible strips are expanded by a surgeon to retract tissue and retain the endoscope in position.

In order to maintain adequate integrity of the gas seal between a cannula and a body cavity it has been known to provide various mechanisms and devices. Typical devices include a medical device for control of enemata disclosed in U.S. Patent No. 3,459,175 to Miller, which provides a hollow pipe adapted for insertion by its forward end through the anal canal into the lower region of a patient's bowel. The device includes an inflatable annular element of resilient flexible material surrounding the pipe and a U-shaped abutment element including legs and a base. The annular element and the abutment element work in concert to prevent flow of fluid from the bowel.

U.S. Patent No. 4,077,412 to Moossun, as well as U.S. Patent No. 4,627,838 to Cross et al., disclose devices to prevent the inadvertent removal or backing off of the cannula during the surgical procedure and provide some sealing properties, and include an inflatable diaphragm member which is inflated once the cannula is positioned in the body cavity to prevent inadvertent removal of the cannula from the incision until the diaphragm is deflated.

U.S. Patent No. 5,002,557 to Hasson, discloses a device which provides a laparoscopic cannula including a truncated cone-shaped collar surrounding the cannula. An expandable member at the device's distal end is used in concert with the collar to capture tissue therebetween. U.S. Patent No. 3,817,251 to Hasson discloses a conical sleeve which may be adjusted to various positions on the cannula to limit insertion of the cannula to specific depths.

The novel tissue gripping device of the present invention obviates the disadvantages encountered in the prior art and provides improvements which are desirable for enhancing the retention properties of the cannula in the body wall. The device of the present invention provides a tissue gripping device which may work in concert with a cannula to retain the cannula in the body wall through the provision of articulated flexible members which extend outwardly to engage the body wall when in their extended position. The device includes means to positively retain the flexible members in an extended position so that the cannula is maintained in the body wall without the requirement of having surgical personnel hold the cannula in place. The device of the present invention further provides a simple device for attaching to the cannula or trocar assembly which ensures desired sealing between the cannula and the body during a surgical procedure. Alternatively, the device may be constructed integrally with a cannula, or as part of a tissue gripping device attached to a cannula. Moreover, the sealing device provides a non-invasive sealing means that will minimize trauma to the tissue surrounding a cannula.

### SUMMARY OF THE INVENTION

The present invention provides a cannula comprising a tissue gripping device and a sealing device. The tissue gripping device of the present invention allows a surgeon to use the cannula in a hands-off manner. The tissue gripping device is positionable about the tubular body portion of the cannula and is slidable about the tubular body portion. The tissue gripping device is selectively engagable between an at rest position and a deployed position. The cannula of the present invention further includes a novel sealing device for use with the cannula or a similar device which works in concert with the tissue gripping device. The sealing device maintains a gas seal externally between the body cavity and the cannula after the body cavity has been insufflated during endoscopic or laparoscopic surgical procedures. The sealing device includes a substantially cylindrical member positioned about the cannula which includes a flange at a distal end positionable against a patient's body. The sealing device of the present invention may be used with various cannulas, or may be constructed integral with a cannula that is part of a trocar assembly. Also, preferably, the sealing device is longitudinally adjustable.

The present invention further provides a sealing device for use with a cannula to inhibit leakage of gas and fluids about an incision. The sealing device includes a body portion having at least one element extending towards a distal end of the cannula. The element includes a flange at a distal end thereof and is positionable against a patients body.

The present invention further provides a sealing device which includes a substantially rigid portion. The sealing device provides enhanced stabilization of the cannula.

In use, the cannula is inserted into a patient, so that the sealing member of the present invention is resiliently engaged against the patient's body. The sealing device helps avoid desufflation of the body cavity by retarding escaping gases at the incision.

It is further contemplated that the device of the present invention be constructed as part of a trocar assembly which includes a cannula, a tissue gripping member, and an obturator.

Further, the present invention provides a tissue gripping apparatus which comprises an outer sleeve concentrically positioned about an inner sleeve. A plurality of articulated arm members integral with the outer sleeve are positioned at a distal end of the outer sleeve. A hinge is located proximal a midpoint of each articulated arm. The articulated arm members further include means to manipulate the arm members to an extended position which situates an arm portion of each arm member at a location proximal the hinge in a substantially perpendicular orientation relative to the inner sleeve. The tissue gripping apparatus of the present invention further includes an improved mechanism for retaining the flexible member in an extended position and a non-extended position.

The present invention further provides a tissue gripping apparatus for use with a cannula. The tissue gripping apparatus of the present invention allows a surgeon to use the cannula in a hands-off manner. The tissue gripping apparatus comprises a cylindrical body portion concentrically supported about a cannula, where the body portion includes a flexible element at its distal end. The flexible element includes a plurality of articulated arm members each having a hinge located proximal of the midpoint of the arm member. The articulated arm member is manipulable between an elongated position in which the arm member is at rest and against the cannula and an extended deployed position which situates an arm portion proximal the hinge in a substantially perpendicular orientation relative to the cannula. The articulated arms are deployed by manipulating the cylindrical body portion of the device. The tissue gripping apparatus of the present invention provides enhanced retention of the surgical apparatus in a patient's body, and includes an improved mechanism for retaining the flexible member in an extended deployed position and a non-extended at rest position.

The present invention further provides a trocar assembly having an obturator and a cannula, in which the cannula of the trocar assembly comprises a tubular body portion having a flexible member concentrically positioned about the tubular body portion. The flexible member includes a plurality of articulated arm members each having a hinge located proximal of a midpoint of the arm member. The articulated arm member is manipulable between a non-extended at rest position and a deployed extended position which situates an arm portion proximal the hinge in a substantially perpendicular orientation relative to the cannula. Further, the cannula of the trocar assembly includes an improved mechanism for retaining the flexible member in an extended deployed position and a non-extended at rest position.

The present invention further includes a method for maintaining a tubular member such as a cannula within an opening in a body. The method comprises positioning an elongated tubular cannula in an incision where a cylindrical member is concentrically positioned about at least a portion of the cannula. The cylindrical member includes a flexible portion disposed at a distal end, and is manipulable between an extended deployed position and a non-extended at rest position by urging the cylindrical member distally to deploy the flexible portion to grip the body wall at the incision.

### BRIEF DESCRIPTION OF THE DRAWINGS

The foregoing features of the present invention will become more readily apparent and may be better understood by referring to the following detailed description of an illustrative embodiment of the tissue gripping device for use with a trocar or cannula, taken in conjunction with the accompanying drawings, in which:
Figure 1 is a perspective view illustrating a tissue gripping apparatus according to a first embodiment of the present invention;
Figure 2 is an exploded perspective view illustrating the tissue gripping apparatus according to a second embodiment of the present invention;
Figure 3 is a perspective view illustrating the tissue gripping apparatus of Figure 2 in an assembled condition in an at rest position;
Figure 4 is a perspective view illustrating the tissue gripping apparatus of Figure 3 in a deployed position;
Figure 5 is a perspective view illustrating a cannula according to the present invention including a tissue gripping device and a sealing device;
Figure 6 is an exploded perspective view illustrating the cannula shown in Figure 5;
Figure 7 is an exploded perspective view illustrating the tissue gripping device and the sealing device shown in Figures 5 and 6;
Figure 8 is a perspective view illustrating a trocar assembly according to another embodiment of the present invention including a tissue gripping device and a sealing device having stabilizing properties;
Figure 9 is a perspective view illustrating another embodiment of a sealing device having stabilizing properties according to the present invention; and
Figure 10 is a bottom view illustrating another embodiment of a sealing device having a pusher guide.

### DETAILED DESCRIPTION OF PREFERRED EMBODIMENTS

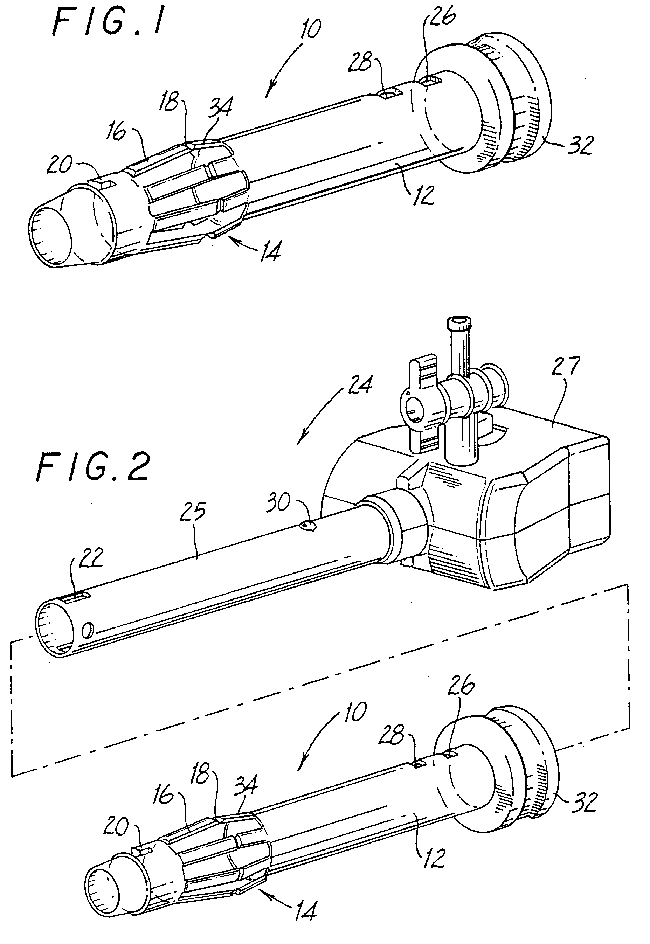

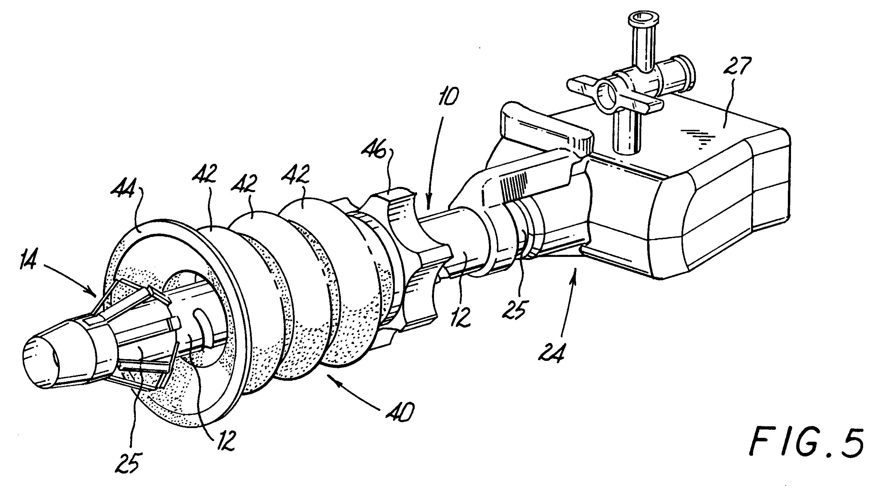

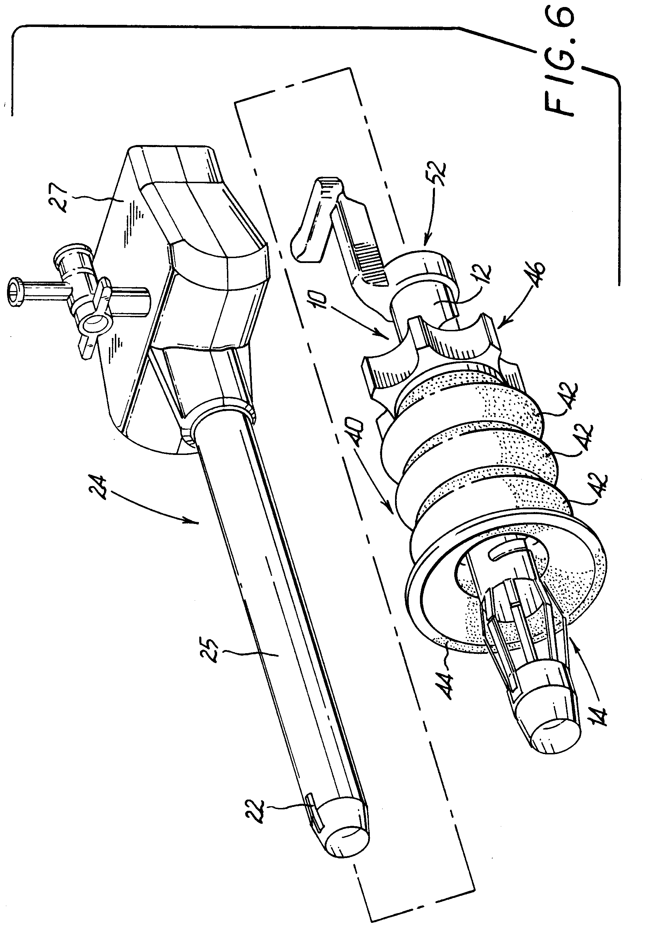

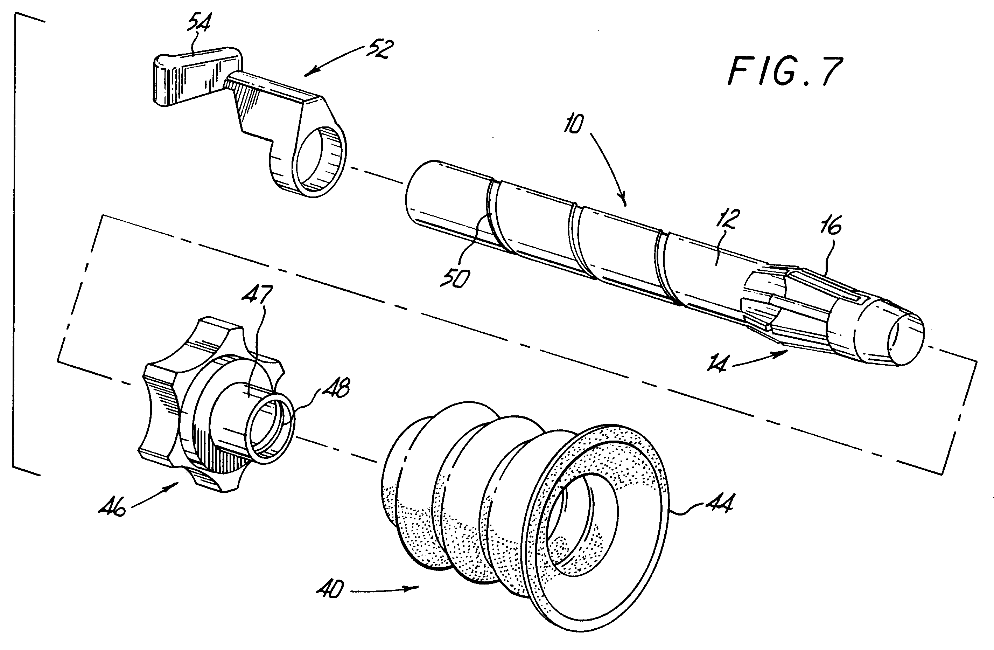

Referring now in specific detail to the drawings, in which like reference numerals identify similar or identical elements, there is shown a tissue gripping apparatus 10 according to the present invention illustrated in Figures 1 through 4. Further, there is shown a sealing device 40 for use with a cannula and for working in concert with the tissue gripping apparatus 10 in accordance with the present invention illustrated in Figures 5 through 7. As illustrated in Figure 1, the tissue gripping apparatus 10 includes a cylindrical body portion 12, and a flexible element 14 having a plurality of substantially parallel arms 16. Each of the arms 16 have a hinge 18 located proximal to a midpoint of each respective arm, preferably each hinge 18 is substantially the same distance from the midpoint of the respective arms 16.

As seen in Figure 2, the cylindrical body portion 12 further includes a resilient member 20 at its distal end comprising a tab which is designed to cooperate with a slot 22 in a cannula 24. The cannula includes a tubular body portion 25 and a valve body portion 27, as discussed below. The cylindrical body portion 12 of apparatus 10 further includes at least two slots 26 and 28 located at a proximal end of the body portion 12. Both slots 26, 28 are preferably at a proximal end of the body portion and are designed to cooperate with a substantially hemispheric surface 30 on the tubular body portion 25 of the cannula 24.

The cylindrical body portion 12, may be constructed, preferably, of a resiliently flexible material such as, for example, a polypropylene material, which is sufficiently resilient to flex over the outwardly directed hemispheric surface 30 on the tubular body portion 25 on the cannula 24. A gripping flange 32 is situated at a proximal end of the cylindrical body portion 12. The flange 32 allows a surgeon to easily move the cylindrical body portion 12 distally to deploy the articulated arms 16, as will be discussed below.

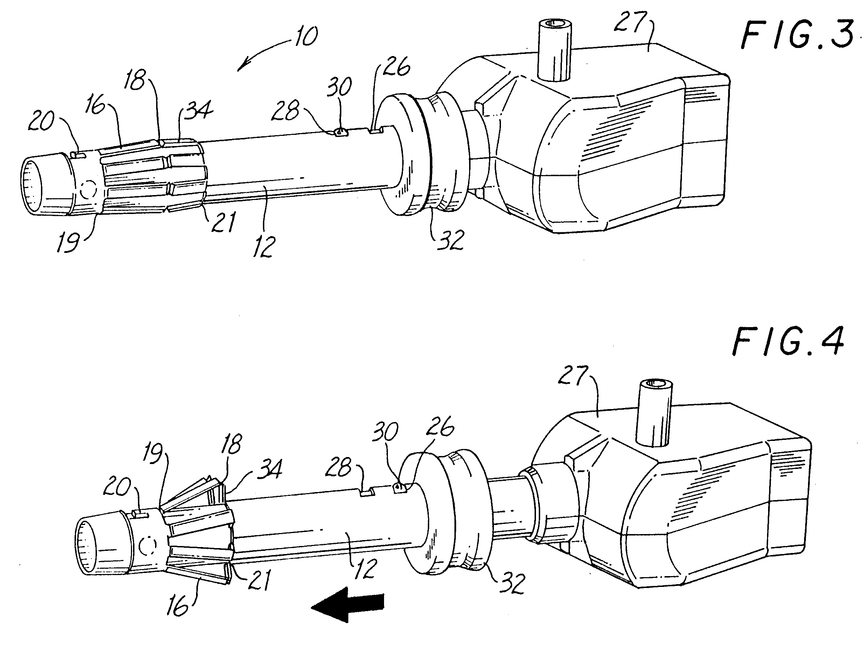

The location of hinge 18, proximal of the midpoint of each respective arm 16, results in an advantageous geometry when the articulated arms 16 are fully deployed when the cylindrical body portion 12 is advanced to its distal-most position, as described below. In particular, when fully deployed, the arm portions 34 on the proximal side of the hinge assume a substantially perpendicular orientation relative to the cannula, as shown in Figure 4. This perpendicular orientation ensures optimal retention of the surgical apparatus in, for example, the abdomen by securingly engaging the inner wall of the abdominal cavity. Generally, from 3 to 12 arms are substantially evenly spaced around the circumference of the body portion 12, and preferably there are from 6 to 10 arms.

Referring to Figure 2, the tissue gripping apparatus 10 of the present invention is used with a cannula 24 of a trocar assembly. The cannula 24 is provided with a substantially hemispheric surface 30 at the proximal end of its tubular member 25. The hemispheric surface 30 cooperates with the slots 26, 28 in the cylindrical body portion 12, such that the distal-most slot 28 cooperates with the hemispheric surface 30 when the tissue gripping apparatus 10 is non-deployed, and the proximal-most slot 26 cooperates with the hemispheric surface 30 when the tissue gripping apparatus 10 is fully deployed; that is, when the articulated arms 16 are in an extended position as shown in Figure 4. The cannula further includes a slot 22 at its distal end designed to cooperate with the resilient tab member 20 at the distal end of the cylindrical body portion.

While it is preferred that the cannula be provided with the hemispheric surface 30 to engage slots 26 and 28 of the apparatus 10, the surface 30 may include an annular ring which would cooperate with an annular groove in apparatus 10, or may include an indentation in cannula body 25 and a corresponding protrusion in body portion 12.

Referring now to Figures 3 and 4, the cylindrical body portion 12 is shown fitted on the cannula 24 according to the present invention. As shown in Figure 3, the articulated arms 16 of the cylindrical body portion 12 are in a non-engaged position and are positioned substantially in contact with the cannula as shown. In this position, the hemispheric surface 30 is cooperating with the distal-most slot 28 on the cylindrical body portion 12.

As seen in Figure 4, the articulated arms 16 are in a deployed position, such that the arm portions 34 of the articulated arms 16 proximal the hinge 18 are in a substantially perpendicular orientation relative to the tubular portion 25 of the cannula 24. The proximal-most slot 26 in the cylindrical body portion 12 is cooperatingly engaged with hemispheric surface 30 to maintain the articulated arms 16 in the engaged position.

In operation, the surgeon uses the trocar assembly, having a pointed obturator (not shown) and inserts the trocar through the body wall of a patient. After the obturator is removed, the surgeon grasps the flanged surface 32 of gripping apparatus 10 and slides the cylindrical body portion 12 distally relative to the stationary cannula 24. The cylindrical body portion 12 material flexes over the hemispheric surface 30 to disengage surface 30 from slot 28, and the body portion 12 is moved until the hemispheric surface 30 enters the proximal-most slot 26. The articulated arms move outwardly as hinges 19 and 21 turn arms 16 and 34 away from body portion 12 through hinge 18, to a fully deployed position. The cooperation between the slots 26, 28 and the hemispheric surface 30 provides an improved mechanism for securing the articulated arms 16 in a deployed engaged and an at rest non-engaged position. The location of the hinge 18 on the articulated arms 16 allows the portion 34 of the articulated arms 16 proximal the hinge 18 to be substantially perpendicular to the tubular portion 25 of the cannula 24. The orientation of the articulated arms provides an enhanced means for restraining the cannula against the body wall in an incision in a patient's body.

Another embodiment of the tissue gripping apparatus 10 of the present invention may include a tube or a cannula having a movable outer sleeve cooperating with an inner sleeve that are not part of a trocar assembly. The inner and outer sleeves operate in a similar fashion to the cylindrical body portion 12 and the cannula 24 described above.

The present invention further comprises a method for gripping tissue which includes the steps of providing an elongated tubular cannula having a cylindrical member positioned about the cannula which includes a plurality of articulated arms. The articulated arms include a hinge positioned as described above. The cannula 24 and cylindrical member 12 cooperate as described above to provide an enhanced tissue gripping method.

Referring to Figures 5-7, a sealing device is provided for use with a tubular member such as a cannula or similar device to deter the escape of gases from the body cavity when, for example, a surgeon is engaging in endoscopic or laparoscopic procedures requiring insufflation of the body cavity.

An embodiment of the sealing device for use with a cannula 24 and working in concert with the tissue gripping apparatus 10 is shown in Fig. 5. The illustrated cylindrical sealing member 40 is slidably positioned about the body portion 12 of the tissue gripping apparatus 10. The cylindrical member 40 is shaped similar to a bellows construction in which a plurality of bulbous portions 42 are integrally molded to each other. The cylindrical member 40 includes a flange 44 at its distal end that is positionable against a patient's body.

It is also contemplated that the bulbous portions 42 of the sealing device 40 be sealingly attached to each other to allow variation of the number of bulbous portions 42 employed. The number of bulbous portions 42 can thereby be selected such that the sealing device 40 will be accordingly lengthened or shortened based upon the number of bulbous portions chosen.

The inner diameter of the cylindrical sealing member 40 is greater at its distal end than the diameter of the body portion 12 of the tissue gripping apparatus 10 to provide room for the body portion 12 to change its angular juxtaposition with respect to the patient's body as the tubular body portion 25 of the cannula 24 is manipulated. Further, the cylindrical sealing member 40 is longitudinally adjustable and resiliently responds when compressed.

A rotatable knob 46 is positioned at the proximal end of the cylindrical member 40. The knob 46 includes a cylindrical body section 47 which is dimensioned and configured to be positioned over the body portion 12 of the tissue gripping apparatus 10. The knob 46 includes a ridge 48 located along the inner circumference of the cylindrical body section 47. The ridge 48 slidably mates with a groove 50 in the body portion 12 of the tissue gripping apparatus 10. The groove 50 helically circumscribes and extends longitudinally about the body portion 12 of the tissue gripping apparatus 10. The ridge 48 and the groove 50 slidably mate such that as the knob 46 is rotated the ridge 48 slides along the groove 50 and is positionable at a desirable location on the body portion 12 of the tissue gripping apparatus 10. The knob 46 may be rotated such that the cylindrical member 40 is compressed against the patient's body such that the flange 44 of the cylindrical member 40 is securely place against the skin of the patient's body, and may compensate for movement of the cannula 24 perpendicular to the body surface.

An actuation member 52 is shown which operates similarly to the flange surface 32 of the tissue gripping apparatus 10 shown in Figures 1-4. However, the actuation member 52 includes surface structure 54 which is configured for actuation by the thumb of the user. The actuation member 52 is moved distally to deploy the substantially parallel arms 16 of the flexible element 14 of the tissue gripping apparatus 10.

It is also envisioned that the cylindrical sealing member 40 comprises a ridge around the inner circumference of its proximal end which directly cooperates with a receiving indentation or groove at the proximal end of the cannula. It is also contemplated that the inner diameter of the cylindrical sealing member at its proximal end may be defined by a neck dimensioned to fit securely about the cannula.

As seen in Fig. 5, the cannula 24 may be part of a trocar assembly, thus enabling the sealing device of the present invention to be used in concert with a tissue gripping apparatus 10. The sealing device 40 may be, for example,

constructed of an elastomeric material, which is preferably an elastomer commercially available under the trademark "SANTOPRENE", manufactured by Monsanto.

In operation, the cylindrical sealing member 40 as shown in Figs. 5-7 is used with the cannula 24 and in concert with the tissue gripping apparatus 10. After the cannula 24 is inserted into the body, the tissue gripping apparatus 10 is actuated into a deployed position by moving the actuation member 52 distally. The cannula 24 is thereby secured in the incision by the extended articulated arms 16 of the tissue gripping apparatus 10.

The cylindrical member 40 of the cylindrical sealing member is then urged towards the patient's body by rotating the knob 46 such that the ridge 48 advances in the groove 50 of the body portion 12 of the tissue gripping apparatus 10. The cylindrical member 40 is compressed a desired amount by continued rotation of the knob 46 after the flange 44 contacts the patient's skin. The flange 44 contacting the patient's skin provides a desired gas seal for maintaining insufflation pressure within the body cavity in the event there is leakage about the tubular portion 25 of the cannula 24 at the incision. The cylindrical sealing member 40 is designed to remain in substantial contact with a patient's body while accommodating the cannula 24 in varying angular positions with respect to a patient's body. More specifically, the cylindrical sealing member 40 includes an inside diameter which allows for angular juxtapositioning of the cannula 24 with respect to a patient's body while maintaining the relationship between the flange 44 and a patient's body.

To remove the cannula 24, the rotatable knob 46 is rotated to move the cylindrical member 40 proximally decompressing the cylindrical member 40 and eventually substantially removing the flange 44 from contact with the patient's skin. The articulated arms 16 of the tissue gripping apparatus 10 are then returned to their at rest position by moving the actuation member 52 proximally. Finally, the entire tissue gripping apparatus 10, cylindrical member 40, and cannula 24 may be withdrawn from the incision.

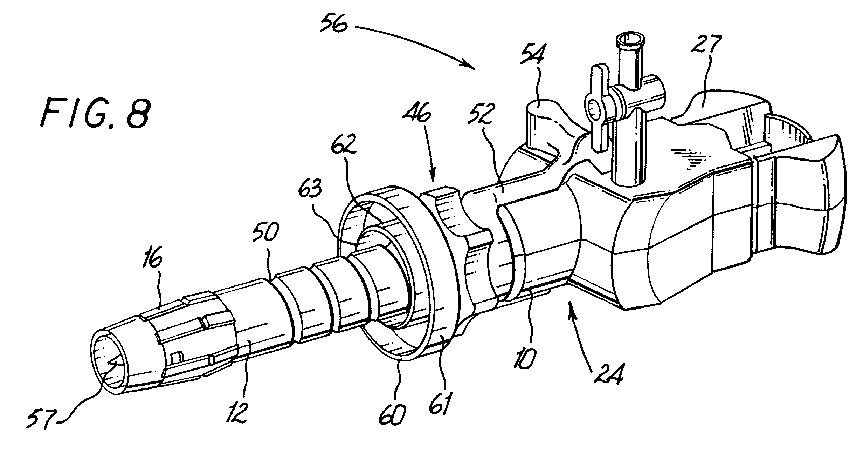

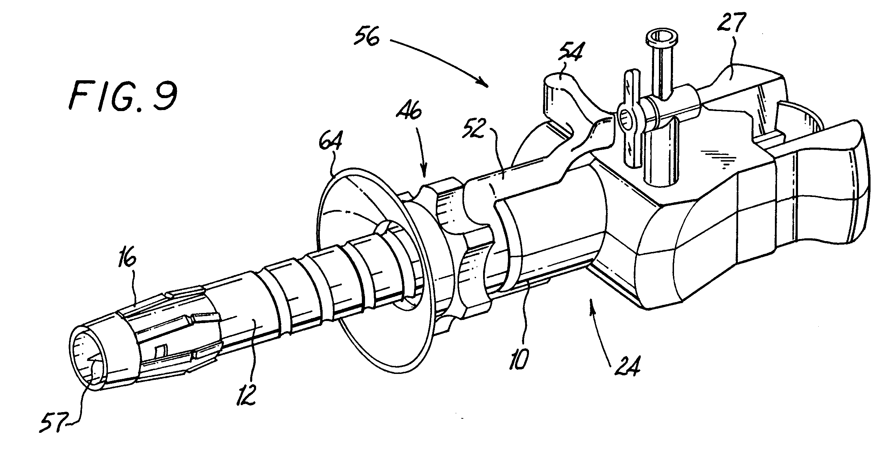

Referring to Figures 8 and 9, alternative embodiments of sealing devices having stabilizing properties are provided for use with a cannula or trocar assembly. The trocar assembly 56, shown in Figs. 8 and 9, includes a cannula 24 working in concert with a tissue gripping apparatus 10 as shown in Figures 1 and 5, and a pointed obturator 57 removably positioned within the tubular body portion 25 of the cannula 24. The illustrated gasket sealing member 58 is slidably positioned about the body portion 12 of the tissue gripping apparatus 10. The gasket sealing member 58 includes an outer ridge 61 positioned circumferentially about the sealing member 58 and extending distally to form a lip 60. A distally extending inner ridge 62 is radially displaced from the outer ridge 62 and includes an inner lip 63. The gasket sealing member 58 is contemplated to be substantially comprised of a rigid material. The rigid nature of the gasket sealing member 58 is such that the sealing member 58 can be placed against a patients skin to enhance stabilization of the cannula 24 positioned through the body wall of the patient.

The gasket sealing member 58 may be used in concert with the tissue gripping apparatus 10, and as part of the trocar assembly 56 shown in Figure 8 in a similar manner as with the previous embodiment shown in Figs. 5-7. Specifically, after the pointed obturator 57 is used to puncture the body cavity, and the cannula 24 is positioned in the body, the tissue gripping apparatus 10 is actuated into a deployed position by moving the actuation member 52 distally. The cannula 24 is thereby substantially secured in the incision by the extended articulated arms 16 of the tissue gripping apparatus 10.

The gasket sealing member 58 is then urged towards the patient's body by rotating knob 46 as in the previous embodiment. The knob 46 is rotated until the lips 60 and 63 of the sealing gasket 58 contact the patient's skin securely. The lips 60 and 63 contacting the patient's skin provide enhanced gas sealing for maintaining insufflation pressure within the body cavity in the event there is leakage about the tubular portion 25 of the cannula 24 at the incision. Further, the rigid nature of the gasket sealing member 58 enhances support of the cannula 24 positioned through an incision in the body cavity. The cannula's increased stability provides greater ease of entry into the cannula by the surgeon, as well as, moderating angular movement of the cannula. This increased stability decreases the likelihood of irritation or trauma around the entry cite of the cannula into the body cavity.

Although the gasket sealing member 58 moderates angular movement of the cannula 24, some angular movement of the cannula 24 is likely and may be desirable. The gasket sealing member 58 is designed to remain in substantial contact with the patient's body while accommodating the cannula 24 in varying angular positions with respect to the patients body. More specifically, the outer ridge 61 and distally extending ridge 62 include an inside diameter which is larger than the outside diameter of the body portion 12 of the tissue gripping apparatus 10. Thus, the inside diameter of the ridge 62 allows for angular juxtapositioning of the cannula 24 with respect to a patient's body while maintaining the relationship between the ridge 62 and the patient's body.

Referring to Figure 9, another sealing member 64 is shown having a substantially abbreviated frustoconical shape. The sealing member 64 can be used in essentially the same manner as the embodiment shown in Figure 8, however, the sealing member 64 shown in Fig. 9 is generally comprised of pliable or flexible materials. The inside diameter of a portion of the sealing member 64 is designed to allow for angular juxtapositioning of the cannula 24. The sealing member may be placed against a patient's body as with the previous embodiment shown in Fig. 8. The flexible nature of the sealing member 64 allows for angular movement of the proximal end of the cannula 24 while providing some stabilization properties for the cannula 24.

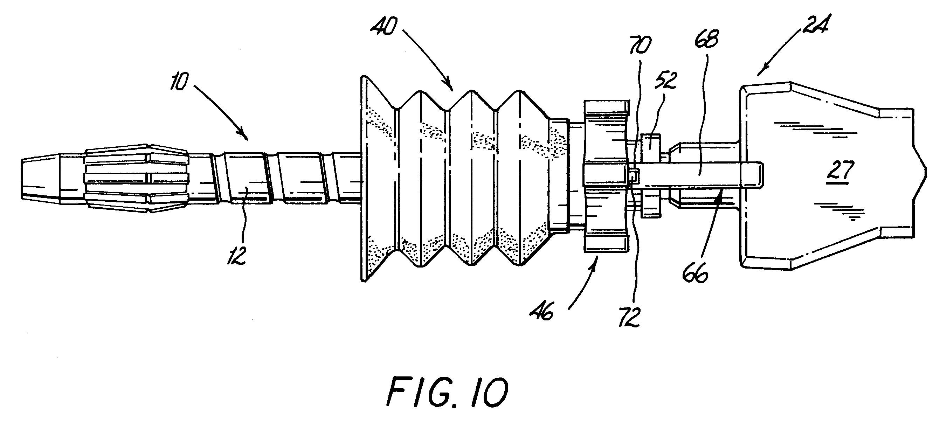

As shown in Figure 10, the cannula 24 may further include a pusher guide 66 which extends transversely across actuation member 52 and includes an elongated body portion 68 and an engagement member 70. The pusher guide 66 cooperates with and engages at least one flange 72 extending from the knob 46. The flange 72 prevents advancement of the knob 46 until after the tissue gripping apparatus 10 has been activated. Specifically, when the tissue gripping apparatus 10 is activated, the cylindrical body portion 12 and the knob 46 engaged thereon are advanced distally such that the flange 72 is advanced out of engagement with engagement member 70. The knob 46 may then be rotated to position the cylindrical member 40.

The claims which follow identify embodiments of the invention additional to those described in detail above.

## Claims

1. A cannula for insertion into an opening in a body wall comprising:
a tubular body portion;
tissue gripping means slidably positionable about said tubular body portion, said tissue gripping means having an outer sleeve slidably positioned about said tubular body portion and including a plurality of articulated arm members positioned at a distal end of said outer sleeve, said arm members having a hinge positioned proximal a midpoint of said arm members, and wherein said articulated arms are manipulable between an at rest position and a deployed position in relation to said body portion; and
sealing means positionable about said tubular body portion, said sealing means inhibiting leakage of gas and fluids about said tubular body portion of said cannula.

2. A cannula according to any preceding claim further comprising retaining means for holding said tissue gripping means in said rest position and said deployed position.

3. A cannula according to any preceding claim wherein said outer sleeve includes an actuation member to move said outer sleeve between said at rest position and said deployed position.

4. A cannula means according to any preceding claim wherein said sealing means is selectively positionable about said tubular body portion.

5. A cannula according to any preceding claim wherein said sealing means is substantially cylindrically shaped and includes a flange at a distal end thereof, said flange being positionable and forming a substantially continuous seal against said body wall.

6. A cannula according to any preceding claim wherein said sealing means is longitudinally deformable.

7. A cannula according to any preceding claim wherein said sealing means includes a plurality of interconnected bulbous portions.

8. A cannula means according to any preceding claim wherein said sealing means is resiliently compressible such that said flange at said distal end substantially contacts said patient's body when said cannula is inserted into said body wall.

9. A cannula according to any preceding claim wherein said sealing means includes a retaining element engaging a groove in said tissue gripping means such that said retaining element is slidably positionable along said groove.

10. A cannula for use in endoscopic surgical procedures comprising:
a tubular body portion;
an outer sleeve slidably positioned about said tubular body portion;
a plurality of articulated arm members positioned at a distal end of said outer sleeve;
at least one hinge positioned proximal of a midpoint of each of said arms, said articulated arms being manipulable between an extended position and a non-extended position such that a portion of each arm proximal each of said hinges is positioned substantially perpendicular relative to said tubular body portion when said arms are in said extended position;
means for retaining said articulated arms in said extended position and said non-extended position; and
sealing means including a substantially cylindrical member longitudinally positionable about said tubular body portion, said substantially cylindrical member being longitudinally deformable, said substantially cylindrical member including a flange at a distal end thereof, said flange being positionable against a patient's body such that said flange forms a substantially continuous seal against the skin of said body wall.

11. A cannula according to any preceding claim wherein said substantially cylindrical member includes an inside diameter allowing angular juxtapositioning of said cannula with respect to said patient's body while said flange remains substantially in contact with said body wall.
